# EUROPEAN PATENT SPECIFICATION

(11) **EP 3 772 334 B1**
(45) Date of publication and mention of the grant of the patent: **14.02.2024**
(21) Application number: 20190022.2
(22) Date of filing: 07.08.2020
(51) Int. Cl.: A61B 10/00

(54) **EXTRACTION DEVICE AND RELATED METHOD FOR SAMPLING MATERIAL**
EXTRAKTIONSVORRICHTUNG UND ZUGEHÖRIGES VERFAHREN ZUR PROBENENTNAHME VON MATERIAL
DISPOSITIF D'EXTRACTION ET PROCÉDÉ ASSOCIÉ POUR MATÉRIAU D'ÉCHANTILLONNAGE

(30) Priority: 08.08.2019 US 201962884499 P
(43) Date of publication of application: 10.02.2021
(73) Proprietor: American Laboratory Products Company, Ltd., Salem, NH 03079 (US)
(72) Inventor: Wisherd, Christopher, Bedford,, New Hampshire 03110 (US); Conley, Sean, Windham, New Hampshire 03087 (US)
(74) Representative: Studio Torta S.p.A.

(56) References cited:
- US-A- 5 554 151
- US-A- 5 869 003
- US-A1- 2005 084 842
- US-A1- 2019 117 332

## Description

### FIELD

The present invention relates generally to the field of extraction devices and methods of using the same. More specifically, the invention relates to a device and a method for the extraction and preparation of a sample, such as a stool sample.

### BACKGROUND

Stool extraction devices exist that allow for the measured sampling of stool. For example, such devices include a dipstick that can be inserted into stool to collect a portion of the stool. The dipstick can then be inserted into a vessel, such as a tube. The vessel includes a cone through which the end of the dipstick that contains the portion of the stool can be inserted. Insertion of the end of the dipstick leaves a predefined amount of the stool on the dipstick, thereby removing an excess amount of the stool.

After passing through the cone, the end of the dipstick with the predefined amount of stool contacts a fluid, such as a buffer solution. After mechanical actuation of the extraction device, such as vigorous shaking, a portion of the predefined amount of the stool comes off of the dipstick and enters the solution. However, some of the predefined amount of the stool remains on the dipstick. Indeed, enough stool remains on the dipstick to affect the accuracy of subsequent analysis that is performed on the prepared solution.

Examples of extraction devices are disclosed in US5869003A, US2005084842A1, and US5554151A. US5869003A discloses an extraction device comprising a tubular housing and a dipstick receiver cap including a cylindrical sleeve or fitment engaging with a dipstick.

It would be desirable to have the ability to remove substantially all of the predefined amount of stool from the dipstick, which would improve the subsequent analysis that is performed on the prepared solution.

### SUMMARY

The invention is defined by independent claim 1. Preferred embodiments are defined in the dependent claims.

One exemplary embodiment of the invention relates to an extraction device having a tube, a dipstick, and a dipstick receiver cap. The tube has a first open end and a second open end generally opposite from the first open end. The dipstick is configured to be inserted into the tube at the first open end. The dipstick has one or more features at a first end. The one or more features are configured to retain a predefined amount of collected material in which the dipstick is inserted prior to inserting the dipstick into the tube. The dipstick receiver cap is removably coupled to the tube at the second open end. The dipstick receiver cap includes a first projection configured to engage the one or more features to substantially remove the predefined amount of collected material from the dipstick.

In one or more embodiments, the one or more features include a helical groove. In one or more embodiments, the projection includes one or more features that engage the helical groove. In one or more embodiments, the one or more features that engage the helical groove include an annular ring. In one or more embodiments, the tube further includes an open-ended cone. In one or more embodiments, the device further includes a dipstick cap removably coupled to the tube at the first open end. The dipstick cap includes a second projection configured to seal the open-ended cone with the dipstick cap coupled to the first open end. In one or more embodiments, a profile of the open-ended cone matches a cross-section of the dipstick, and the open-ended cone is configured to remove excess collected material from the dipstick upon the dipstick being inserted through the open-ended cone. In one or more embodiments, the dipstick is removably coupled to the second projection of the dipstick cap.

Another exemplary embodiment of the invention relates to an extraction device having a vessel, an object, a first feature, and a second feature. The vessel is configured to hold a fluid. The object has a first end configured to be inserted into and retain material external to the vessel. The first feature is within the vessel and is configured to leave a predefined amount of the material on the first end of the object as the first end interfaces with the first feature. The second feature is within the vessel and is configured to assist in transferring the predefined amount of the material into the fluid as the first end of the object interfaces with the second feature.

In one or more embodiments, the first end of the object includes a groove that retains the predefined amount of the material, and the second feature is a complementary ridge that fits into the groove. In one or more embodiments, the groove is helical. In one or more embodiments, the vessel comprises a tube with a first aperture and a second aperture generally opposite from the first aperture. The device further includes a first cap removably coupled to the first aperture, and a second cap removably coupled to the second aperture. The second feature is integral with the second cap. In one or more embodiments, the first feature includes an open-ended cone configured to remove an excess amount of the material as the first end of the object is inserted there through, thereby leaving the predefined amount of material. In one or more embodiments, the open-ended cone is integral with the vessel. In one or more embodiments, the first feature at least partially defines a reservoir within the vessel configured to contain the fluid, and the second feature is within the reservoir.

Another exemplary embodiment of the invention relates to a method of sampling a predefined amount of collected material on a dipstick. The method includes inserting a first end of the dipstick through an open end of a tube. The method includes further engaging one or more features on the first end of the dipstick that are retaining the predefined amount of the collected material with a projection within the tube to substantially remove the predefined amount of the collected material from the one or more features.

In one or more embodiments, the one or more features include a helical groove. In one or more embodiments, the projection includes one or more features that engage the helical groove. In one or more embodiments, the one or more features that engage the helical groove include an annular ring. In one or more embodiments, the method further includes forming the predefined amount of the collected material by inserting the first end of the dipstick through an aperture defined by a cone within the tube to remove excess material from the collected material.

It is to be understood that both the foregoing general description and the following detailed description are exemplary and explanatory only and are not restrictive of the invention as claimed.

### BRIEF DESCRIPTION OF THE DRAWINGS

These and other features, aspects, and advantages of the present invention will become apparent from the following description, appended claims, and the accompanying exemplary embodiments shown in the drawings, which are briefly described below.
FIG. 1 is a perspective view of an extraction device, according to one embodiment of the present disclosure.
FIG. 2 is a side view of the extraction device of FIG. 1, according to one embodiment of the present disclosure.
FIG. 3 is a cross-sectional view along the line 3-3 of FIG. 2 of the extraction device of FIG. 1, according to one embodiment of the present disclosure.
FIG. 4 is an exploded view of the extraction device of FIG. 1, according to one embodiment of the present disclosure.
FIG. 5 is another exploded view of the extraction device of FIG. 1, according to one embodiment of the present disclosure.
FIG. 6A illustrates a step for collecting material using the extraction device of FIG. 1, according to one embodiment of the present disclosure.
FIG. 6B illustrates another step for collecting material using the extraction device of FIG. 1, according to one embodiment of the present disclosure.
FIG. 6C illustrates another step for collecting material using the extraction device of FIG. 1, according to one embodiment of the present disclosure.
FIG. 7A illustrates a step for dispersing material within fluid of the extraction device of FIG. 1, according to one embodiment of the present disclosure.
FIG. 7B illustrates another step for dispersing material within fluid of the extraction device of FIG. 1, according to one embodiment of the present disclosure.
FIG. 7C illustrates another step for dispersing material within fluid of the extraction device of FIG. 1, according to one embodiment of the present disclosure.
FIG. 7D illustrates another step for dispersing material within fluid of the extraction device of FIG. 1, according to one embodiment of the present disclosure.
FIG. 7E illustrates another step for dispersing material within fluid of the extraction device of FIG. 1, according to one embodiment of the present disclosure.

While the invention is susceptible to various modifications and alternative forms, a specific embodiment thereof has been shown by way of example in the drawings and will herein be described in detail. It should be understood, however, that it is not intended to limit the invention to the particular forms disclosed, but, on the contrary, the intention is to cover all modifications, equivalents, and alternatives falling within the scope of the invention as claimed in the appended set of claims.

### DETAILED DESCRIPTION

As discussed above, an issue with conventional extraction devices is that stool samples do not fully come off of the dipstick. As a result, conventional extraction devices do not obtain the desired dilution ratio of the stool to the fluid, among other issues. The extraction device of the present disclosure solves the issues of conventional extraction devices by including one or more features that substantially remove material collected on the dipstick.

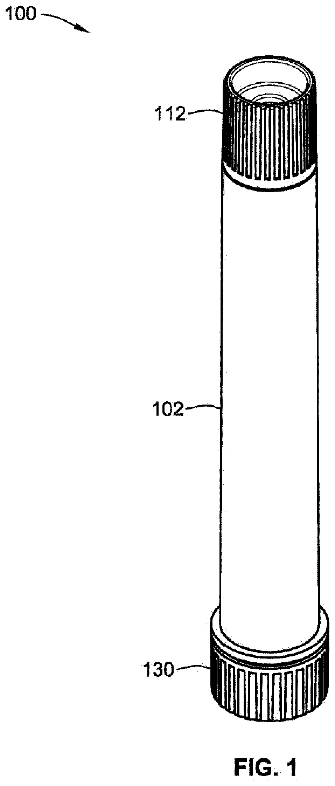

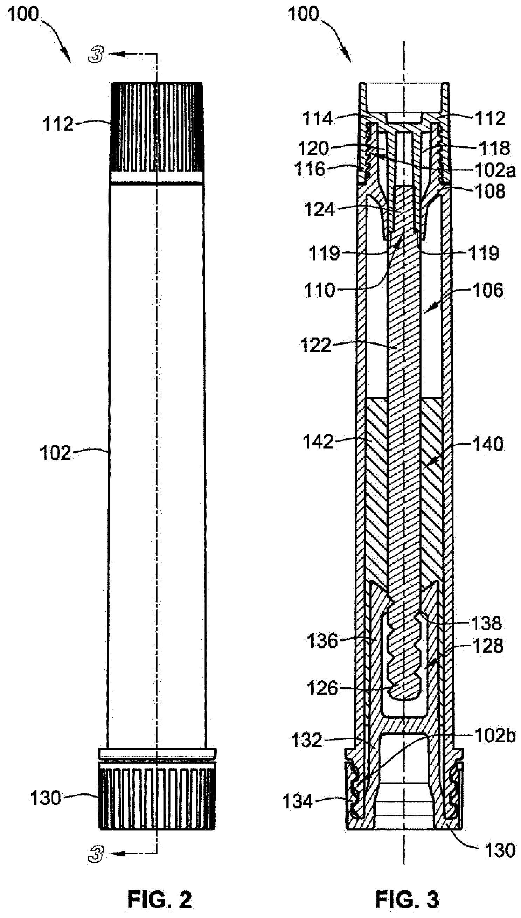

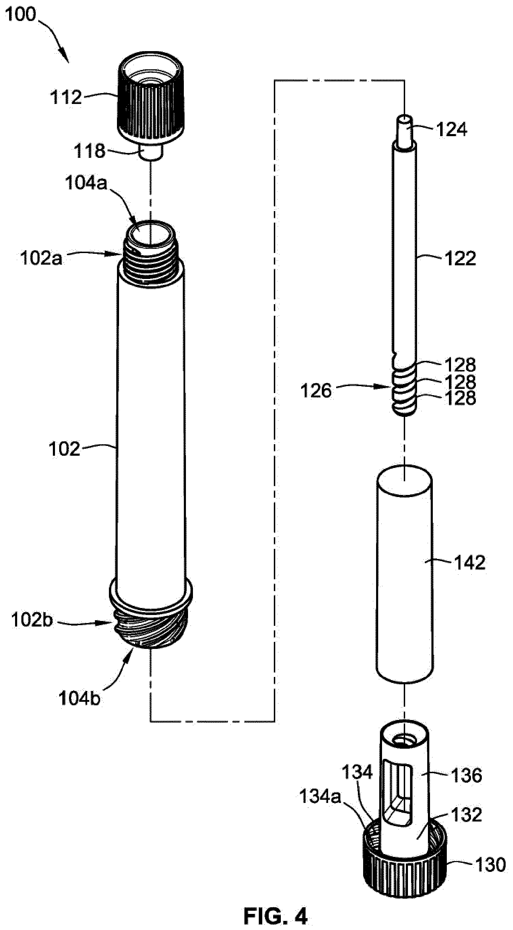

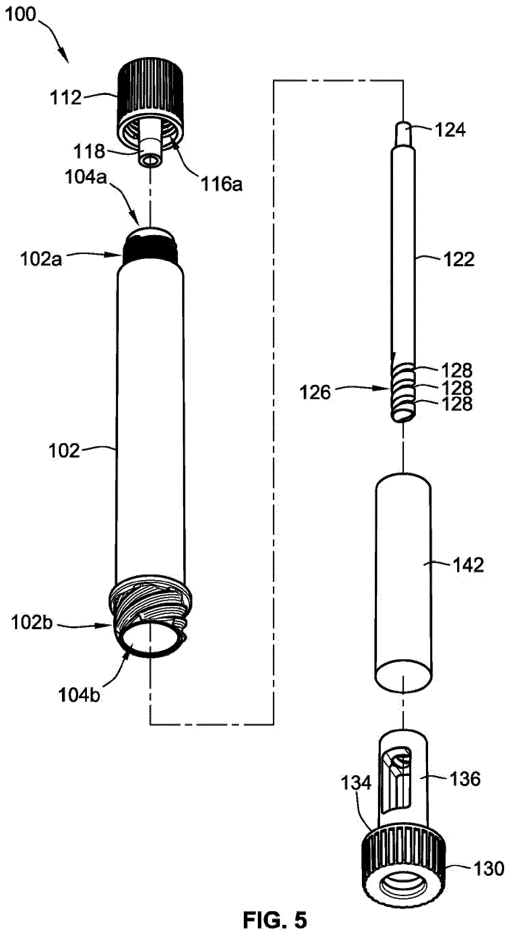

Referring to FIGS. 1 through 5, illustrated is a perspective view (FIG. 1), a side view (FIG. 2), a cross-sectional view (FIG. 3, along line 3-3 of FIG. 2), and exploded views (FIGS. 4 and 5) of an extraction device 100, according to one embodiment of the present disclosure. The extraction device 100 includes a tube 102. The tube 102 is elongated and has a first open end 104a and a second open end 104b, generally opposite from the first open end 104a. Although referred to and illustrated as a tube, the tube 102 can be any type of vessel that can contain a sample prepared from material that is inserted into the tube 102.

In one or more embodiments, the tube 102 can include a threaded portion 102a at the open end 104a and/or a threaded portion 102b at the open end 104b. The two threaded portions 102a and 102b can be configured for accepting threaded caps, as further discussed below.

Within the interior 106 of the tube 102 is a cone 108. The cone 108 is open-ended and defines an aperture 110. As described further below, the cone 108 and the aperture 110 interface with an object that is inserted into the tube 102 for removing excess material from the object. Accordingly, in one or more embodiments, the aperture 110 defined by the cone 108 has a cross-sectional profile that matches that of the object inserted therethrough.

Although referred to and illustrated as a cone, the cone 108 can be any one or more features configured to remove excess material from an object as the object interfaces with the one or more features. For example, the one or more features can be a series of projections that interface with an object as the object is inserted into the tube 102, such as series of baffles arranged offset from each other along the length of the tube 102. Thus, the cone 108 can instead be any feature(s) configured to leave a predefined amount of a material on a first end of an object as the first end engages with the one or more features.

The extraction device 100 further includes a dipstick cap 112. The dipstick cap 112 is configured to releasably couple to the first open end 104a of the tube 102 to seal the first open end 104a. In one or more embodiments, the dipstick cap 112 includes a dipstick cap body 114, a dipstick cap plug 116, and a dipstick cap projection 118.

The dipstick cap plug 116 seals the first open end 104a so as to prevent material (e.g., fluid) from entering or escaping from the tube 102 with the dipstick cap 112 coupled to the tube 102. In one or more embodiments, the dipstick cap plug 116 can include a threaded portion 116a to assist in securing the dipstick cap 112 to the tube 102. Similarly, the tube 102 at the open end 104a can include the threaded portion 102a to assist in securing the dipstick cap 112 to the tube 102. Alternatively, in one or more embodiments the dipstick cap plug 116 and the tube 102 can lack the threaded portions 116a and 102a, respectively. Instead, the dipstick cap 112 can couple to the tube 102 by, for example, a pressure fitting or other mechanical arrangement, such as a snap fitting.

The projection 118 extends from the dipstick cap body 114 into the tube 102. In one or more embodiments, the projection 118 is configured to engage the cone 108 through the aperture 110 to form a seal 119 within the tube 102. The tube 102 between the seal 119 and the dipstick cap body 114 forms a reservoir 120. As discussed below, the reservoir 120 can be configured to hold excess material within the tube 102 that is removed from an object inserted into the tube and through the aperture 110.

The extraction device 100 further includes a dipstick 122. A first end 124 of the dipstick 122 releasably couples to the projection 118. The dipstick 122 can couple to the projection 118 at the first end 124 via a press fitting or some other form of mechanical fitting, such as complementary threaded portions.

A second end 126 of the dipstick 122, generally opposite from the first end 124, includes one or more features 128. The one or more features 128 are configured to retain material into which the dipstick 122 is inserted, prior to the dipstick 122 being inserted into the tube 102. More specifically, the one or more features 128 are configured to retain a predefined amount of the material into which the second end 126 of the dipstick 122 is inserted after inserting the second end 126 of the dipstick through the cone 108. In one or more embodiments, the predefined amount of the material can be, for example, about 15 milligrams (mg).

In one or more embodiments, the features 128 can be one or more grooves, including one or more helical grooves, such as to form an auger. However, the features 128 can be any type of feature that can retain material into which the dipstick 122 is inserted for sampling the material, as discussed further below.

Although described as a dipstick, the term dipstick refers to any object, such as a wand, a stick, a rod, or the like, that can be inserted into material to be sampled and retain at least part of the material. Thus, the term dipstick is not meant to be limiting unless otherwise noted.

The device 100 further includes a receiver cap 130. The receiver cap 130 is configured to releasably couple to the second open end 104b of the tube 102 to seal the second open end 104b. In one or more embodiments, the receiver cap 130 includes a receiver cap body 132, a receiver cap plug 134, and a receiver cap projection 136. The receiver cap plug 134 seals the second open end 104b so as to prevent material (e.g., fluid) from entering or escaping from the tube 102 with the receiver cap 130 coupled to the tube 102.

In one or more embodiments, the receiver cap plug 134 can include a threaded portion 134a to assist in securing the receiver cap 130 to the tube 102. Similarly, the tube 102 at the open end 104b can include the threaded portion 102b to assist in securing the receiver cap 130 to the tube 102. Alternatively, in one or more embodiments the receiver cap plug 134 and the tube 102 can lack the threaded portions 134a and 102b, respectively. Instead, the receiver cap 130 can couple to the tube 102 by, for example, a pressure fitting or other mechanical arrangement, such as a snap fitting.

The projection 136 extends from the receiver cap body 132 into the tube 102. The projection 136 is configured to engage the dipstick 122 when the dipstick 122 is inserted into the tube 102 through the first open end 104a. For example, the dipstick 122 and the projection 136 can be configured so that the second end 126 of the dipstick 122 extends fully into the projection 136 when the dipstick 122 is fully inserted into the tube 102. The dipstick 122 can be fully inserted into the tube 102 when the dipstick 122 is coupled to the dipstick cap 112 and the dipstick cap 112 is coupled to the tube 102.

The projection 136 further includes one or more features 138 that engage with the one or more features 128 of the dipstick 122 to remove, or at least substantially remove, all of the material retained by the one or more features 128. In one or more embodiments, the one or more features 138 can be an annular detent or ring configured to fit within the helical grooves on the second end 126 of the dipstick 122, as discussed further below. In one or more embodiments, the one or more features 138 can be a complementary ridge that fits the one or more features 128 on the second end 126 of the dipstick 122.

The tolerance between the one or more features 128 on the second end 126 of the dipstick 122 and the one or more features 138 on the projection 136 of the receiver cap 130 can be configured such that the one or more features remove all, or substantially all, of the material from the dipstick 122. In one or more embodiments, substantially all of the material can be removed from the dipstick 122 such that the resulting prepared sample of the material within the tube 102 provides accurate results when subjected to subsequent analysis. In one or more embodiments, substantially all of the material can be removed from the dipstick 122 such that the desired ratio of material to fluid 142 within the tube 102 can obtained.

The tube 102 between the seal 119 and the receiver cap body 132 forms a second reservoir 140. The second reservoir 140 holds a fluid 142. The fluid 142 can be, for example, a solution for dispersing the material collected on the dipstick 122, as further discussed below.

In one or more embodiments, the tube 102 can be pre-filled with the fluid 142. Alternatively, the tube 102 can be filled with the fluid 142 after inserting the dipstick 122 within the tube 102 (either with or without a sample retained on the dipstick 122). The amount of fluid can vary depending on various factors, such as the size of the extraction device 100. In one or more embodiments, the amount of the fluid 142 can be about 1.5 milliliters (ml) of fluid. With about 1.5 ml of fluid and about 15 mg of material removed from the features 128 of the dipstick 122, the dilution of the material in the fluid can be about 1:100. In one or more embodiments, the fluid 142 can be a universal extraction buffer.

In one or more embodiments, the tube 102 can come with the open ends 104a and 104b sealed with one or more seals, such as a foil seal, prior to the dipstick cap 112 and/or receiver cap 130 being coupled to the tube 102. In one or more embodiments, the act of coupling the dipstick cap 112 and/or receiver cap 130 onto the tube 102 can cause the foil seals to break. Otherwise, the foil seals can be removed prior to the dipstick cap 112 and/or the receiver cap 130 being coupled to the tube 102.

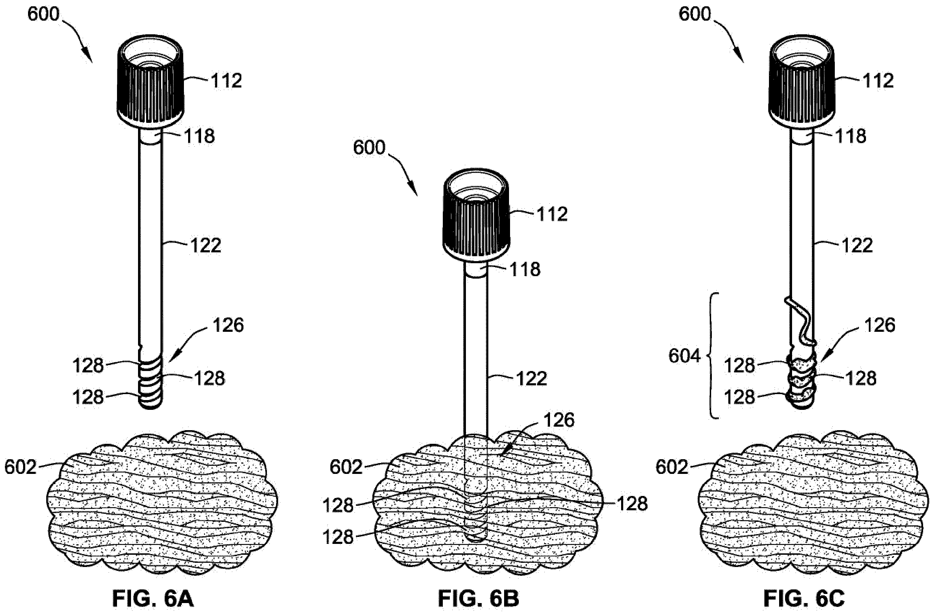

Referring to FIGS. 6A through 6C, illustrated are steps for collecting material 602 on the dipstick 122, according to one embodiment of the present disclosure. The dipstick 122 is illustrated as being coupled to the dipstick cap 112 so as to form a dipstick assembly 600. However, in one or more embodiments, the dipstick 122 can be de-coupled from the dipstick cap 112 while collecting the material on the dipstick 122.

Referring to FIG. 6A, the dipstick assembly 600 is brought over the material 602. In one or more embodiments, the material 602 can be stool. However, although the present disclosure focuses primarily on stool being the material 602 collected by the dipstick 122, the material 602 can be any type of material that is to be diluted within a fluid for preparing a solution for sampling.

Referring to FIG. 6B, the second end 126 of the dipstick 122 is inserted into the material 602. The dipstick 122 is inserted into the material 602 such that the features 128 on the second end 126 of the dipstick 122 are fully submerged in the material 602. This promotes the material 602 being collected within all of the features 128. In one or more embodiments, a single insertion of the dipstick 122 into the material 602 may be all that is needed to fill the features 128 with the material 602. Alternatively, the dipstick 122 may be repeatedly inserted into the material 602 to fill the features 128 with the material 602.

Referring to FIG. 6C, the dipstick 122 is removed from the material 602. As illustrated, the features 128 are full of collected material 604. Further, additional collected material 604 may be on the second end 126 of the dipstick 122, such as between the features 128, above the features 128, and/or below the features 128.

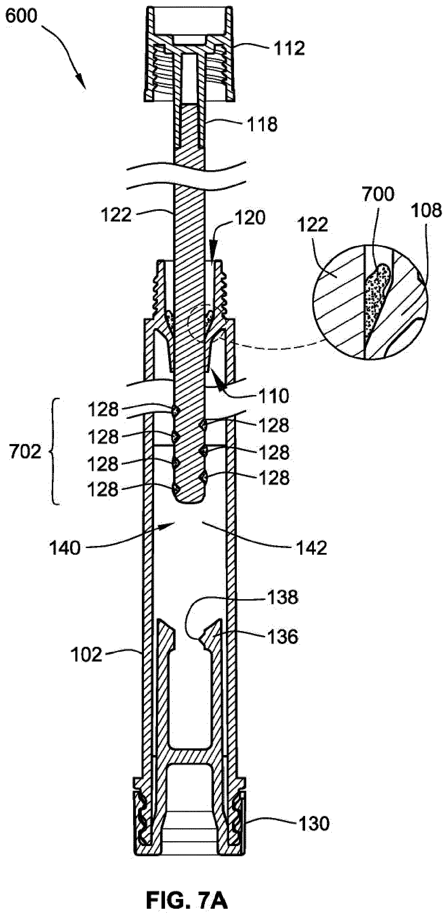

Referring to FIGS. 7A through 7E, illustrated are steps for dispersing the collected material 604 within the fluid 142 of the extraction device 100, according to one embodiment of the present disclosure.

Referring to FIG, 7A, the second end 126 of the dipstick 122 is inserted into the first open end 104a of the tube 102, and then further through the aperture 110 of the cone 108. The aperture 110 is sized so as to permit the dipstick 122 through but remove excess material 700 from the collected material 604 on the dipstick 122. In one or more embodiments, the excess material 700 can also come from the features 128, such as if the features 128 have retained more of the material 602 than configured for sampling. The excess material 700 is collected on the cone 108 within the reservoir 120. The seal 119 between the projection 118 and the cone 108 keeps the excess material 700 within the reservoir 120.

Remaining on the second end 126 of the dipstick 122 within the features 128 is a predefined amount 702 of the collected material 604. Thus, the size and/or shapes of the cone 108, the aperture 110, the dipstick 122, and the features 128 are configured to retain the predefined amount 702 of the collected material 604 within the features 128 after the second end 126 of the dipstick 122 is inserted through the aperture 110 of the cone 108. As discussed above, the predefined amount 702 of the material 602 can be, for example, about 15 mg. However, the amount can vary depending on, for example, the size of the extraction device 100, the quantity of predefined amount 702 needed for sampling, etc.

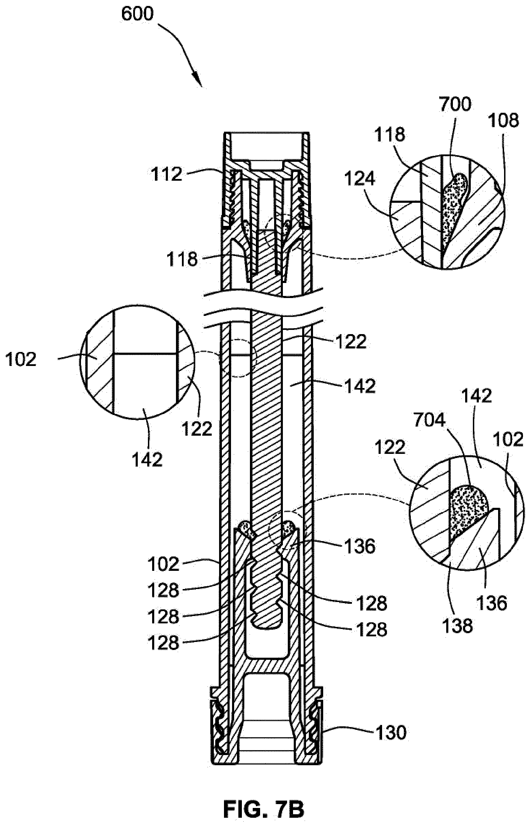

Referring to FIG. 7B, the second end 126 of the dipstick 122 is further inserted into the tube 102 as so to engage the projection 136 of the receiver cap 130. Further, the one or more features 128 on the second end 126 of the dipstick 122 engage the one or more features 138 on the projection 136. Engagement of the one or more features 128 with the one or more features 138 fully, or at least substantially, removes the predefined amount 702 of the collected material 604 from the one or more features 128. Thereafter, the predefined amount 702 of the collected material 604 is fully dispersed within the fluid 142, or at least partially dispersed within the fluid 142 and partially retained on the projection 136. Thus, the one or more features 138 are configured to assist in transferring the predefined amount 702 of the collected material 604 off of the dipstick 122 and into the fluid 142 as the first end 124 of the dipstick 122 interfaces with the one or more features 138.

As discussed above, the tolerances of the one or more features 128 and the one or more features 138 are configured to fully, or at least substantially, remove the predefined amount 702 of the collected material 604. In one or more embodiments, the one or more features 138 can be configured to be slightly larger than that one or more features 128 so as to fully or substantially wipe, scrape, or otherwise remove the predefined amount 702 of the collected material 604 from the one or more features 128. For example, the one or more features 128 can be a helical groove. The one or more features 138 can be an annular ring on the projection 136. The annular ring can be slightly larger than the helical groove but configured such that the annular ring fully engages the helical groove and forces the predefined amount 702 of the collected material 604 out from the helical groove.

In one or more embodiments, the one or more features 138 can be formed of a resilient or semi-resilient material that assists in removing or substantially removing the predefined amount 702 of the collected material 604 from the one or more features 138 by flexing the fit within the one or more features 128.

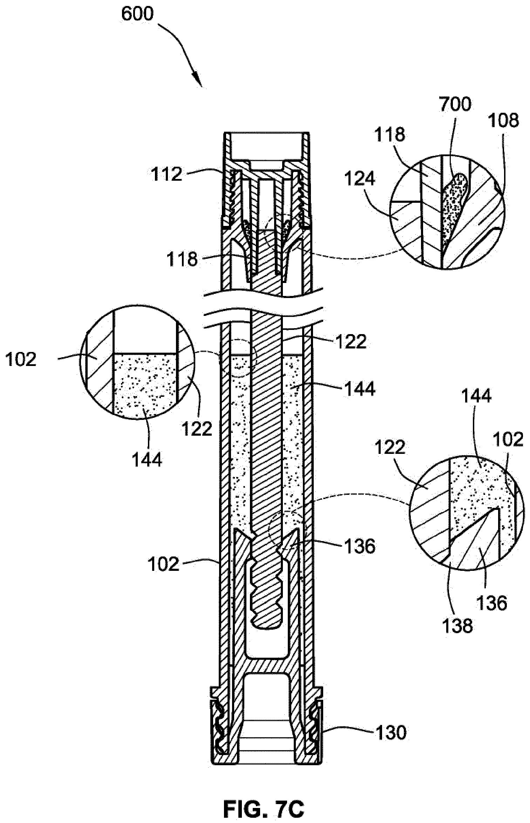

Referring to FIG. 7C, illustrated is the extraction device 100 with the predefined amount 702 of the collected material 604 dispersed within the fluid 142, forming the sample fluid 144. The extraction device 100 illustrated in FIG. 7B can undergo vigorous shaking that aids the predefined amount 702 of the collected material 604 in dispersing into the fluid 142. For example, the extraction device 100 can be placed on a shaker table (e.g., 800 rpm 4 mm Orbital) and/or under centrifugation (e.g., 3000 relative centrifugal force (RCF)) to assist in dispersing the predefined amount 702 of the collected material 604 into the fluid 142.

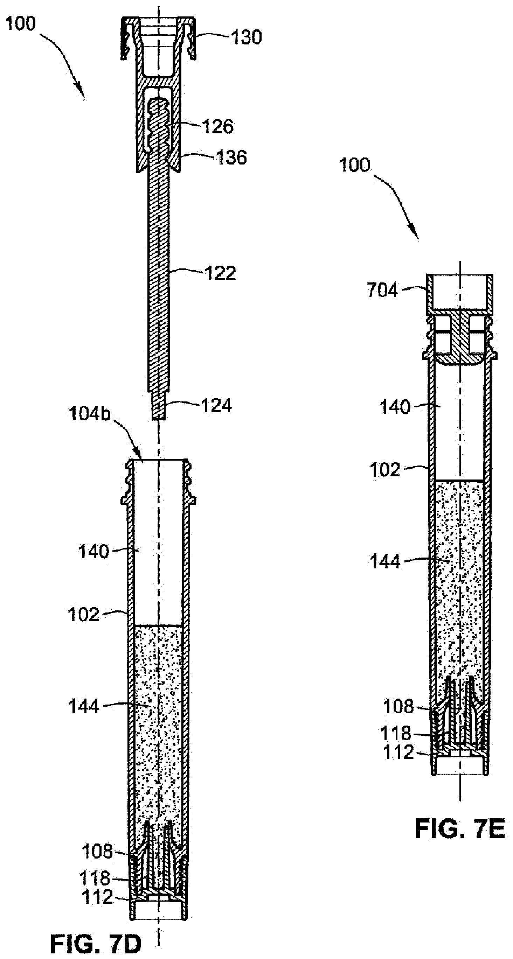

Referring to FIG. 7D, after preparing the sample fluid 144, the tube 102 can be flipped over and the receiver cap 130 can be removed from the tube 102. The removal of the receiver cap 130 simultaneously removes the dipstick 122 by the dipstick 122 de-coupling from the dipstick cap projection 118 of the dipstick cap 112. For example, the dipstick 122 can be configured to release from the pressure fitting with the dipstick cap projection 118, unscrew from the dipstick cap projection 118, or otherwise decouple from the dipstick cap projection 118 depending on how the dipstick 122 is coupled to the dipstick cap projection 118. Removal of the dipstick 122 allows for further downstream processing of the sample fluid 144 in the tube 102, such as allowing a pipette to be inserted into the tube 102 for removal of the sample fluid 144. Insertion of the pipette can be done manually, such as by a lab technician inserting the pipette, or automatically, such as by a device that is attached to or otherwise has access to the tube 102 after removal of the receiver cap 130 and the dipstick 122 inserting the pipette.

Referring to FIG. 7E, a seal cap 704 can be coupled to the second end 104b of the tube 102 to seal the sample fluid 144 in the tube 102. The extraction device 100 can then be used, for example, downstream in an analysis of the sample fluid 144. For example, the analysis can be an immunoassay, such as an enzyme-linked immunosorbent assay (ELISA), a lateral flow immunoassay, a multiplex assay, or another type of analysis that uses the sample fluid 144 with the dispersed predefined amount 702 of collected material 604. For example, one single extraction can be used to determine the levels of 13 biomarkers in multiple gastroenterology research areas including: calprotectin, pancreatic elastase, alpha-1 antitrypsin, secretory IgA, lysozyme, beta defensin 2, bile acids, anti-gliadin antibodies, anti-transglutaminase antibodies, EDN/EPX, albumin, hemoglobin, and hemoglobin/haptoglobin complex. Because of the presence of the projection 136 with the one or more features 138 engaging the one or more features 128 on the dipstick 122, the desired amount of the collected material 604 is dispersed within the fluid 142, which provides accurate results in the downstream analysis.

Based on the foregoing, the extraction device 100 disclosed herein simplifies sample processing and replaces manual weighing while still providing accurate results, unlike conventional extraction devices.

Each of these embodiments and obvious variations thereof is contemplated as falling within the scope of the invention as claimed in the appended set of claims. Moreover, the present concepts expressly include any and all combinations and sub-combinations of the preceding elements and aspects.

As utilized herein, the terms "approximately," "about," "substantially", and similar terms are intended to have a broad meaning in harmony with the common and accepted usage by those of ordinary skill in the art to which the subject matter of this disclosure pertains. It should be understood by those of skill in the art who review this disclosure that these terms are intended to allow a description of certain features described and claimed without restricting the scope of these features to the precise numerical ranges provided. Accordingly, these terms should be interpreted as indicating that insubstantial or inconsequential modifications or alterations of the subject matter described and claimed are considered to be within the scope of the invention as recited in the appended claims.

It should be noted that the term "exemplary" as used herein to describe various embodiments is intended to indicate that such embodiments are possible examples, representations, and/or illustrations of possible embodiments (and such term is not intended to connote that such embodiments are necessarily extraordinary or superlative examples).

The terms "coupled," "connected," "attached," and the like as used herein mean the joining of two members directly or indirectly to one another. Such joining may be stationary (e.g., permanent) or moveable (e.g., removable or releasable). Such joining may be achieved with the two members or the two members and any additional intermediate members being integrally formed as a single unitary body with one another or with the two members or the two members and any additional intermediate members being attached to one another.

References herein to the positions of elements (e.g., "top," "bottom," "above," "below," etc.) are merely used to describe the orientation of various elements in the Figures. It should be noted that the orientation of various elements may differ according to other exemplary embodiments, and that such variations are intended to be encompassed by the present disclosure.

## Claims

1. An extraction device comprising:
a tube (102) having a first open end (104a) and a second open end (104b) generally opposite from the first open end (104a);
a dipstick (122) configured to be inserted into the tube (102) at the first open end (104a), the dipstick (122) having one or more features (128) at a first end (126), the one or more features (128) configured to retain collected material in which the dipstick (122) is inserted prior to inserting the dipstick (122) into the tube (102); and
a dipstick receiver cap (130) removably coupled to the tube (102) at the second open end (104b), the dipstick receiver cap (130) including a first projection (136),
**characterised by** said collected material being a predefined amount of collected material and the first projection (136) including one or more features (138) configured to form a complementary fit within the one or more features (128) of the dipstick (122) to substantially remove the predefined amount of collected material from the one or more features (128) of the dipstick (122).

2. The extraction device of claim 1, wherein the one or more features (128) of the dipstick (122) comprise a helical groove.

3. The extraction device of claim 2, wherein the one or more features (138) of the first projection (136) are configured to engage the helical groove.

4. The extraction device of claim 3, wherein the one or more features (138) configured to engage the helical groove comprise an annular ring.

5. The extraction device of claim 1, wherein the tube (102) further comprises an open-ended cone (108).

6. The extraction device of claim 5, further comprising:
a dipstick cap (112) removably coupled to the tube (102) at the first open end (104a), the dipstick cap (112) including a second projection (118) configured to seal the open-ended cone (108) with the dipstick cap (112) coupled to the first open end (104a).

7. The extraction device of claim 6, wherein a profile of the open-ended cone (108) matches a cross-section of the dipstick (122), and the open-ended cone (108) is configured to remove excess collected material from the dipstick (122) upon the dipstick being inserted through the open-ended cone (108).

8. The extraction device of claim 6, wherein the dipstick (122) is removably coupled to the second projection (118) of the dipstick cap (130).

9. The extraction device of claim 1, wherein the collected material is stool.

10. The extraction device of claim 5, wherein the open-ended cone (108) is integral with the tube (102).

11. A method of sampling a predefined amount of collected material on a dipstick (122), comprising:
inserting a first end (126) of the dipstick (122) through an open end (104a) of a tube (102); and
engaging one or more features (128) on the first end (126) of the dipstick (122) that are retaining the predefined amount of the collected material with one or more features (138) of a projection (136) of a dipstick receiver cap (130) coupled to the tube (102), **characterised by** the one or more (138) of the projection (136) forming a complementary fit within the one or more features (128) of the dipstick (122) to substantially remove the predefined amount of the collected material from the one or more features (128) of the dipstick (122).

12. The method of claim 11, wherein the one or more features (128) of the dipstick (122) comprise a helical groove.

13. The method of claim 12, wherein that the one or more features (138) of the projection (136) engage the helical groove.

14. The method of claim 13, wherein the one or more features (138) of the projection (136) that engage the helical groove comprise an annular ring.

15. The method of claim 11, further comprising:
forming the predefined amount of the collected material by inserting the first end (126) of the dipstick (122) through an aperture (110) defined by a cone (108) within the tube (102) to remove excess material from the collected material.

## Patentansprüche

1. Eine Entnahmevorrichtung, umfassend:
ein Rohr (102) mit einem ersten offenen Ende (104a) und einem dem ersten offenen Ende (104a) allgemein gegenüberliegenden zweiten offenen Ende (104b);
einen Messstab (122), der so konfiguriert ist, dass er an dem ersten offenen Ende (104a) in das Rohr (102) eingeführt werden kann,
wobei der Messstab (122) ein oder mehrere Merkmale (128) an einem ersten Ende (126) aufweist, wobei das eine oder die mehreren Merkmale (128) konfiguriert sind, um gesammeltes Material zurückzuhalten, in das der Messstab (122) vor dem Einführen des Messstabs (122) in das Rohr (102) eingeführt wird; und
eine Messstabaufnahmekappe (130), die abnehmbar mit dem Rohr (102) am zweiten offenen Ende (104b) gekoppelt ist, wobei die Messstabaufnahmekappe (130) einen ersten Vorsprung (136) aufweist, **gekennzeichnet durch** das gesammelte Material, das eine vordefinierte Menge an gesammeltem Material ist, und den ersten Vorsprung (136), der ein oder mehrere Merkmale (138) enthält, die so ausgebildet sind, dass sie einen entsprechenden Formschluss in dem einen oder den mehreren Merkmalen (128) des Messstabs (122) bilden, um im Wesentlichen die vordefinierte Menge an gesammeltem Material von dem einen oder den mehreren Merkmalen (128) des Messstabs (122) zu entfernen.

2. Die Entnahmevorrichtung nach Anspruch 1, wobei das eine oder die mehreren Merkmale (128) des Messstabs (122) eine schraubenförmige Rille umfassen.

3. Entnahmevorrichtung nach Anspruch 2, wobei das eine oder die mehreren Merkmale (138) des ersten Vorsprungs (136) so gestaltet sind, dass sie in die schraubenförmige Nut eingreifen.

4. Entnahmevorrichtung nach Anspruch 3, wobei das eine oder die mehreren Merkmale (138), die so gestaltet sind, dass sie in die schraubenförmige Nut eingreifen, einen ringförmigen Ring umfassen.

5. Die Entnahmevorrichtung nach Anspruch 1, wobei das Rohr (102) ferner einen offenendigen Kegel (108) umfasst.

6. Die Entnahmevorrichtung nach Anspruch 5 ferner umfassend:
eine Messstabkappe (112), die abnehmbar mit dem Rohr (102) am ersten offenen Ende (104a) gekoppelt ist, wobei die Messstabkappe (112) einen zweiten Vorsprung (118) aufweist, der so konfiguriert ist, dass er den Konus (108) mit offenem Ende abdichtet, wobei die Messstabkappe (112) mit dem ersten offenen Ende (104a) gekoppelt ist.

7. Entnahmevorrichtung nach Anspruch 6, wobei ein Profil des offenendigen Kegels (108) mit einem Querschnitt des Messstabs (122) übereinstimmt und der offenendige Kegel (108) so konfiguriert ist, dass er überschüssiges gesammeltes Material von dem Messstab (122) entfernt, wenn der Messstab durch den offenendigen Kegel (108) eingeführt wird.

8. Entnahmevorrichtung nach Anspruch 6, wobei der Messstab (122) abnehmbar mit dem zweiten Vorsprung (118) der Messstabkappe (130) verbunden ist.

9. Die Entnahmevorrichtung nach Anspruch 1, wobei das gesammelte Material Stuhl ist.

10. Entnahmevorrichtung nach Anspruch 5, wobei der offenendige Kegel (108) einstückig mit dem Rohr (102) ist.

11. Verfahren zur Probenahme einer vordefinierten Menge an gesammeltem Material auf einem Messstab (122), umfassend:
Einführen eines ersten Endes (126) des Messstabs (122) durch ein offenes Ende (104a) eines Rohrs (102); und
Ineinandergreifen von einem oder mehreren Merkmalen (128) an dem ersten Ende (126) des Messstabs (122), die die vordefinierte Menge des gesammelten Materials zurückhalten, mit einem oder mehreren Merkmalen (138) eines Vorsprungs (136) einer Messstabaufnahmekappe (130), die mit dem Rohr (102) verbunden ist,
**gekennzeichnet durch** den einen oder die mehreren Merkmale (138) des Vorsprungs (136), die eine formschlüssige Passung in dem einen oder den mehreren Merkmalen (128) des Messstabs (122) bilden, um im Wesentlichen die vordefinierte Menge des gesammelten Materials von dem einen oder den mehreren Merkmalen (128) des Messstabs (122) zu entfernen.

12. Verfahren nach Anspruch 11, wobei das eine oder die mehreren Merkmale (128) des Messstabs (122) eine schraubenförmige Rille umfassen.

13. Verfahren nach Anspruch 12, wobei das eine oder die mehreren Merkmale (138) des Vorsprungs (136) in die schraubenförmige Rille eingreifen.

14. Verfahren nach Anspruch 13, wobei das eine oder die mehreren Merkmale (138) des Vorsprungs (136), die in die schraubenförmige Nut eingreifen, einen ringförmigen Ring umfassen.

15. Verfahren nach Anspruch 11, ferner umfassend:
Ausbilden der vordefinierten Menge des gesammelten Materials durch Einführen des ersten Endes (126) des Messstabs (122) durch eine Öffnung (110), die durch einen Kegel (108) innerhalb des Rohrs (102) definiert ist, um überschüssiges Material aus dem gesammelten Material zu entfernen.

## Revendications

1. Dispositif d'extraction comprenant :
un tube (102) ayant une première extrémité ouverte (104a) et une deuxième extrémité ouverte (104b) généralement opposée à la première extrémité ouverte (104a) ;
une tige-jauge (122) configurée pour être insérée dans le tube (102) au niveau de la première extrémité ouverte (104a),
la tige-jauge (122) ayant une ou plusieurs caractéristiques (128) au niveau d'une première extrémité (126), les une ou plusieurs caractéristiques (128) étant configurées pour retenir la matière collectée dans laquelle la tige-jauge (122) est insérée avant d'insérer la tige-jauge (122) dans le tube (102) ; et
un capuchon de réception de tige-jauge (130) couplé, de manière amovible, au tube (102) au niveau de la deuxième extrémité ouverte (104b), le capuchon de réception de tige-jauge (130) comprenant une première saillie (136),
**caractérisé par** ladite matière collectée qui est une quantité prédéfinie de matière collectée et la première saillie (136) qui comprend une ou plusieurs caractéristiques (138) configurées pour former un ajustement complémentaire dans les une ou plusieurs caractéristiques (128) de la tige-jauge (122) afin de retirer sensiblement la quantité prédéfinie de matière collectée des une ou plusieurs caractéristiques (128) de la tige-jauge (122).

2. Dispositif d'extraction selon la revendication 1, dans lequel les une ou plusieurs caractéristiques (128) de la tige-jauge (122) comprennent une rainure hélicoïdale.

3. Dispositif d'extraction selon la revendication 2, dans lequel les une ou plusieurs caractéristiques (138) de la première saillie (136) sont configurées pour mettre en prise la rainure hélicoïdale.

4. Dispositif d'extraction selon la revendication 3, dans lequel les une ou plusieurs caractéristiques (138) configurées pour mettre en prise la rainure hélicoïdale comprennent une bague annulaire.

5. Dispositif d'extraction selon la revendication 1, dans lequel le tube (102) comprend en outre un cône à extrémité ouverte (108).

6. Dispositif d'extraction selon la revendication 5, comprenant en outre :
un capuchon de tige-jauge (112) couplé, de manière amovible, au tube (102) au niveau de la première extrémité ouverte (104a), le capuchon de tige-jauge (112) comprenant une seconde saillie (118) configurée pour sceller le cône à extrémité ouverte (108) avec le capuchon de tige-jauge (112) couplé à la première extrémité ouverte (104a).

7. Dispositif d'extraction selon la revendication 6, dans lequel un profil du cône à extrémité ouverte (108) correspond à une section transversale de la tige-jauge (122) et le cône à extrémité ouverte (108) est configuré pour retirer l'excès de matière collectée de la tige-jauge (122) après que la tige-jauge a été insérée dans le cône à extrémité ouverte (108).

8. Dispositif d'extraction selon la revendication 6, dans lequel la tige-jauge (122) est couplée, de manière amovible, à la deuxième saillie (118) du capuchon de tige-jauge (130).

9. Dispositif d'extraction selon la revendication 1, dans lequel la matière collectée est des selles.

10. Dispositif d'extraction selon la revendication 5, dans lequel le cône à extrémité ouverte (108) est solidaire avec le tube (102).

11. Procédé d'échantillonnage d'une quantité prédéfinie de matière collectée sur une tige-jauge (122), comprenant les étapes consistant à :
insérer une première extrémité (126) de la tige-jauge (122) par une extrémité ouverte (104a) d'un tube (102) ; et
mettre en prise une ou plusieurs caractéristiques (128) sur la première extrémité (126) de la tige-jauge (122) qui retiennent la quantité prédéfinie de la matière collectée, avec une ou plusieurs caractéristiques (138) d'une saillie (136) d'un capuchon de réception de tige-jauge (130) couplées au tube (102),
**caractérisé par** les une ou plusieurs caractéristiques (138) de la saillie (136) qui forment un ajustement complémentaire dans les une ou plusieurs caractéristiques (128) de la tige-jauge (122) pour retirer sensiblement la quantité prédéfinie de la matière collectée des une ou plusieurs caractéristiques (128) de la tige-jauge (122).

12. Procédé selon la revendication 11, dans lequel les une ou plusieurs caractéristiques (128) de la tige-jauge (122) comprennent une rainure hélicoïdale.

13. Procédé selon la revendication 12, dans lequel les unes ou plusieurs caractéristiques (138) de la saillie (136) mettent en prise la rainure hélicoïdale.

14. Procédé selon la revendication 13, dans lequel les une ou plusieurs caractéristiques (138) de la saillie (136) qui mettent en prise la rainure hélicoïdale comprennent une bague annulaire.

15. Procédé selon la revendication 11, comprenant en outre l'étape consistant à :
former la quantité prédéfinie de la matière collectée en insérant la première extrémité (126) de la tige-jauge (122) dans une ouverture (110) définie par un cône (108) à l'intérieur du tube (102) afin de retirer la matière en excès de la matière collectée.
